# EUROPEAN PATENT APPLICATION

(11) **EP 0 838 458 A1**
(43) Date of publication of application: **29.04.1998**
(21) Application number: 96914446.8
(22) Date of filing: 27.05.1996
(51) Int. Cl.: C07D 257/04

(54) **NOVEL REAGENT FOR TETRAZOLE SYNTHESIS AND PROCESS FOR PRODUCING TETRAZOLES THEREWITH**

(30) Priority: 26.05.1995 JP 128576/95
(71) Applicant: CHUGOKU KAYAKU KABUSHIKI KAISHA, Kure 737 (JP); WAKUNAGA SEIYAKU KABUSHIKI KAISHA, Osaka-Shi Osaka 532 (JP)
(72) Inventor: TOKUHARA, Ginro, Kure-shi, Hiroshima 737 (JP); YAMAGUCHI, Tadashi, Seaki-gun, Hiroshima 737-23 (JP); IWASAKI, Tetsuya, Aki-gun, Hiroshima 736 (JP)
(74) Representative: Hansen, Bernd, Dr. Dipl.-Chem.
(86) International application number: JP9601413
(87) International publication number: WO9637481

(57) **Abstract**

Analkali metal azide and zinc chloride are used in combination as a tetrazole forming agent when producing 1H-tetrazoles of the formula (II): 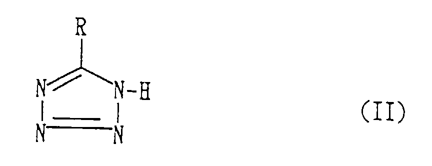 (where R is any substituent) from carbonitriles of the formula (I):

R-CN (I)

(where R has the same meaning as defined above). The tetrazole forming agent permits many kinds of solvents to be used and can in principle be used with any carbonitriles. In addition, the use of inexpensive zinc chloride leads to cost reduction.

## Description

### Technical Field

This invention relates to a novel reagent for the synthesis of tetrazoles and a process for producing tetrazoles using the same.

### Background Art

Described below are the methods heretofore known as techniques for converting carbonitrile forms into tetrazoles.
(A) The method described in J. Ame. Chem. Soc., vol. 80, 1958, pp. 3908 - 3911. This reference describes a method in which alkylnitriles or arylnitriles are reacted with ammonium chloride/sodium azide or alkylamine hydrochlorides/sodium azide in a solvent such as dimethylformamide (DMF) or diethyl sulfoxide to synthesize corresponding tetrazoles.
(B) The method described in J. Org. Chem., vol. 56, 1991, pp. 2395 - 2400. This reference teaches a method of synthesizing tetrazole compounds having steric hindrance. Briefly, carbonitrile compounds are reacted with trialkyltin azides in a solvent such as toluene or xylene to synthesize tetrazole compounds having steric hindrance.
(C) The method described in Japanese Patent Publication No. Hei 6-73028. This publication describes a method in which cyanobenzene compounds are reacted with compounds of the formula (R)₃SnN₃ (where R is an alkyl group having 7 - 18 carbon atoms) to produce tetrazole benzene compounds.
(D) The method described in Chemische Berichte, vol. 89, No. 11 (1956), pp. 2648 - 2653. This reference discloses a method in which nitrile compounds are reacted with aluminum chloride/sodium azide in tetrahydrofuran (THF) to synthesize tetrazole compounds.

With those nitrile compounds of the formula R-CN (where R is any substituent) in which the group R is bulky enough to cause steric hindrance, the method (A) has achieved a very low yield or has required a very long reaction time to provide a satisfactory yield.

The above-described methods (B) and (C) are known to be capable of efficient synthesis of tetrazole compounds having steric hindrance; however, the trialkyltin azides are expensive and lower alkyltin azides such as the tributyltin azide which is used in the method (B) have sufficiently high vapor pressure to be toxic; hence, even these methods have been unsuitable for use as industrial manufacturing processes.

Further, aluminum chloride used as a reagent in the production of tetrazole compounds by the method (D) is only compatible with limited solvents (typically tetrahydrofuran) because of its nature and hence does not have general applicability.

### Disclosure of the Invention

The present invention provides tetrazole forming agents which are safe, inexpensive and which yet have such high flexibility in use that they are industirally advantageous. The invention also provides an industrial process for the production of tetrazole compounds using said tetrazole forming agents.

In view of the problems of the aforementioned prior art, the present inventors conducted intensive studies on industrially salient, novel tetrazole forming agents. As a result, they found that the desired tetrazole compounds could be produced in an industrially safe manner by using ZnCl₂ in combination with an alkali metal azide such as NaN₃, and this finding has led to the accomplishment of the present invention.

Briefly, the present inveniton relates to a novel and useful reagent for the synthesis of 1H-tetrazoles of the formula (II): 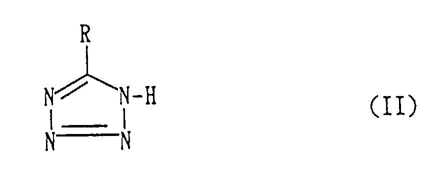 (where R is any substituent) from carbonitriles of the formula (I):

R - CN (I)

(where R has the same meaning as defined above), said reagent being characterized by comprising a metal alkali metal azide and zinc chloride. The invention also relates to a process for producing 1H-tetrazole compounds of the formula (II): 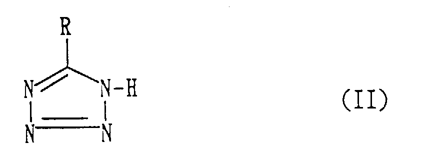 (where R is any substituent), characterized by reacting carbonitrile compounds of the formula (I):

R - CN (I)

(where R has the same meaning as defined above) with an alkali metal azide in the presence of catalytic zinc chloride.

### Best Mode for Carrying out the Invention

The alkali metal azides to be used as a component of the tetrazole forming agent of the invention include lithium azide, sodium azide, potassium azide, rubidium azide and cesium azide, among which sodium azide is particularly preferred.

The nitrile compounds of the formula (I) from which 1H-tetrazole compounds can be synthesized using the tetrazole forming agent of the invention (i.e., zinc chloride/alkali metal azide) are not limited to any particular compounds and the tetrazole forming aqents of the invention can in principle be applied to any nitrile forms. In other words, R in the formula (I) represents any substituent. Examples of R include: optionally substituted alkyl groups (e.g., methyl, ethyl, n-propyl, i-propyl, n-butyl, s-butyl, i-butyl, t-butyl, n-pentyl, n-hexyl, n-heptyl, n-octyl, n-nonyl, n-decyl, undecyl, pentadecyl and heptadecyl groups); optionally substituted alkenyl groups (e.g., vinyl, l-propenyl, allyl, l-butenyl, 2-butenyl and 3-butenyl groups); optionally substituted alkynyl groups (e.g., ethynyl and propargyl groups); optionally substituted alkoxy groups (e.g., methoxy, ethoxy, n-propoxy, i-propoxy, n-butoxy and t-butoxy groups); optionally substituted alkylthio groups (e.g., methylthio and ethylthio groups); optionally substituted alkylsulfonyl groups (e.g., metyl group); optionlly substituted alkylsulfinyl groups (e.g., methylsulfinyl group); optionally substituted alkylamino groups (e.g., methylamino and ethylamino groups); optionally substituted alkenyloxy groups (e.g., vinyloxy, 1-propenyloxy and allyloxy groups); optionally substituted alkenylthio groups (e.g., vinylthio, l-propenylthio and allylthio groups); optionally substituted alkoxycarbonyl groups (e.g., methoxycarbonyl, ethoxycarbonyl, n-propoxycarbonyl, i-propoxycarbonyl, n-butoxycarbonyl and t-butoxycarbonyl groups); optionally substituted aryl groups (e.g., phenyl, o-tolyl, m-tolyl, p-tolyl, o-biphenylyl, m-biphenylyl, p-biphenylyl, α-naphthyl and β-naphthyl groups); optionally substituted aryloxy groups (e.g., phenoxy, o-tolyloxy, m-tolyloxy, p-tolyloxy, o-biphenylyloxy, m-biphenylyloxy, p-biphenylyloxy, α-naphthyloxy and β-naphthyloxy groups); optionally substituted aralkyl groups (e.g., benzyl and phenethyl groups); optionally substituted arylalkenyl groups (e.g., styryl and cinnamyl groups); and optionally substituted heterocyclic groups (e.g., 2-pyridyl, 3-pyridyl and 4-pyridyl groups).

Examples of the groups that may be substituted in the substituents listed above include lower alkyl groups having 1 - 4 carbon atoms (e.g., methyl and ethyl groups), lower alkoxy groups having 1 - 4 carbon atoms (e.g., methoxy and ethoxy groups), halogen atoms (e.g., F, Cl, Br and I), hydroxy group, amino group, nitro group, cyano group, trifluoromethyl group and carboxy group. Either one or more than one group may be substituted in the substituents listed above.

Stated more specifically, R is selected from the group consisting of: hydrogen; a straight-chained or branched, optionally substituted alkyl group having 1 - 4 carbon atoms; a straight-chained or branched, optionally substituted alkoxy group having 1 - 4 carbon atoms; a straight-chained or branched, optionally substituted alkylsulfonyl group having 1 - 4 carbon atoms; a phenyl group; a monosubstituted phenyl group of the following formula: 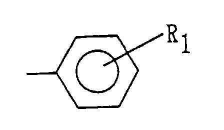 (where R₁ is selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a disubstituted phenyl group of the following formula: 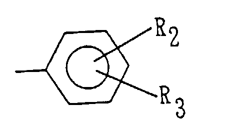 (where R₂ and R₃ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a, straight-chained or branched alkylsulfonyl group having 1 - 4 carbon atoms, a nitro group, an amino group and a carboxyl group); a 4'-methylbiphenyl-2-yl group; a group of the formula: 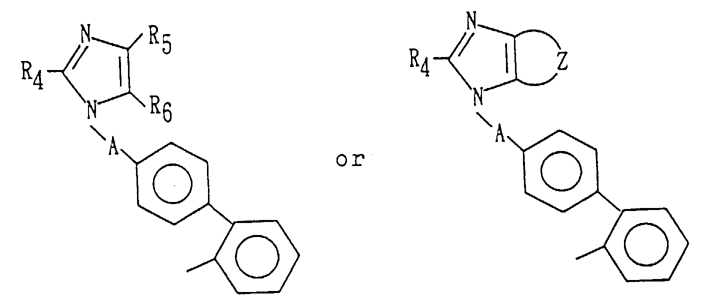 (where A is either O, NH or CH₂; Z is a divalent group selected as having either one of the following formulae: 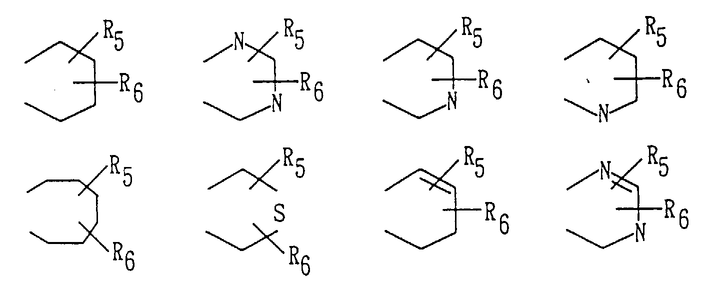 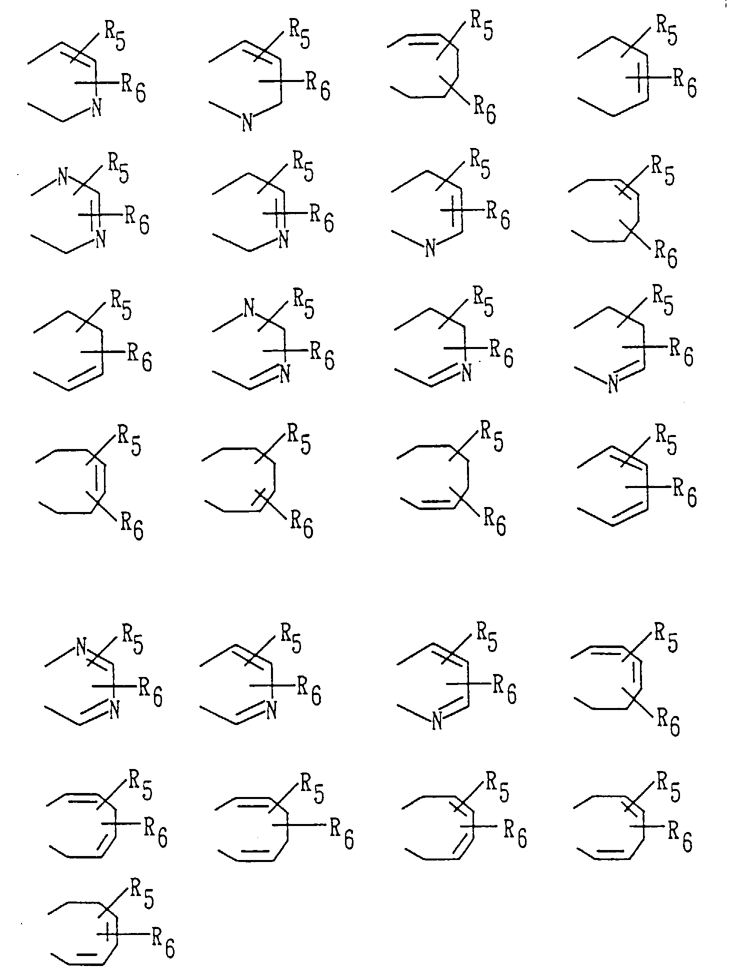 R₄, R₅ and R₆ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a group of the following formula: 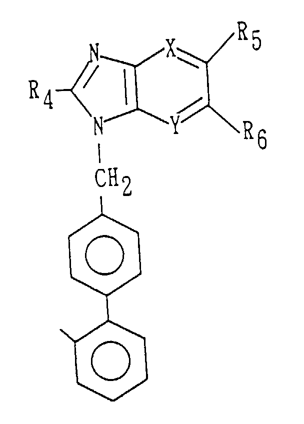 (where X and Y are independently selected from among CH and N, and R₄, R₅ and R₆ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); either one of the groups represented by the following formulae A:

### Formulae A

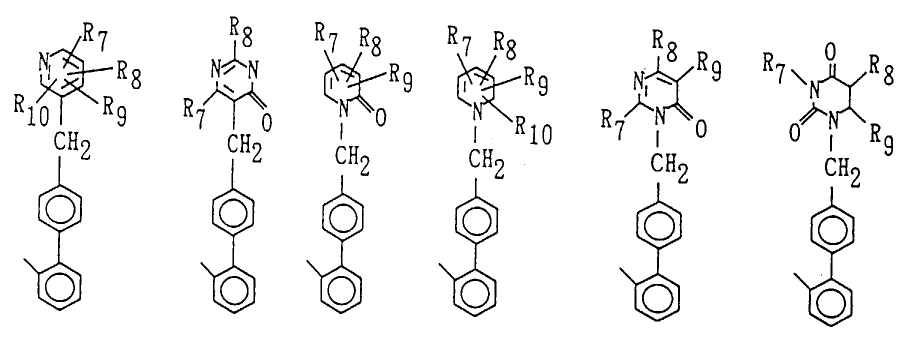

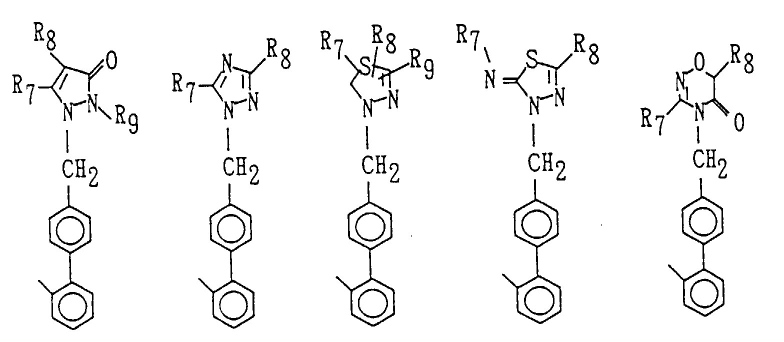

(where R₇, R₈, R₉ and R₁₀ are independently selected from the group consisting of hydrogen, a straight-chained or branched alkyl group having 1 - 4 carbon atoms (e.g., methyl, ethyl, n-propyl, isopropyl, n-butyl or t-butyl), a hydroxyl group, a phenyl group, a halogen atom which is either one of F, Cl, Br and I, a trifluoroacetyl group, a pentafluoropropionyl group, a straight-chained or branched alkoxycarbonyl group (e.g., methoxycarbonyl or ehtoxycarbonyl), a phenyl-substituted straight-chained or branched alkyl group having 1 - 4 carbon atoms (e.g., benzyl or phenethyl) and a hydroxyl-substituted straght-chained or branched alkyl group (e.g., hydroxymethyl or 2-hydroxyethyl)); either one of the groups represented by the following formulae B:

### Formulae B

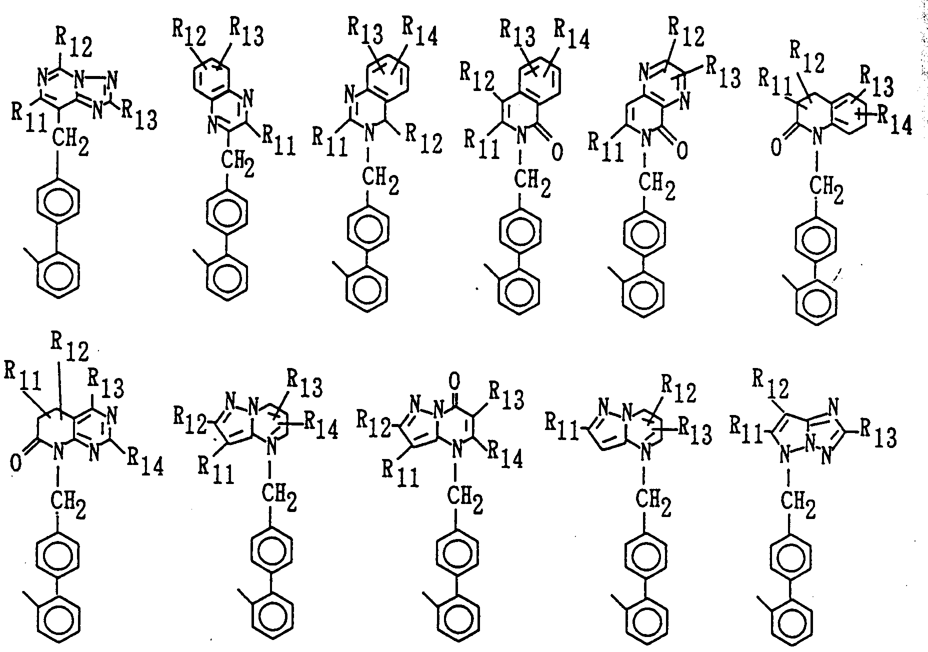

(where R₁₁, R₁₂, R₁₃ and R₁₄ are independently selected from the group consisting of hydrogen, a straight-chained or branched alkyl group having 1 - 4 carbon atoms (e.g., methyl, ethyl, n-propyl, isopropyl, n-butyl or t-butyl), a hydroxyl group, a phenyl group, a halogen atom which is either one of F, Cl, Br and I, a trifluoroacetyl group, a pentafluoropropionyl group, a straight-chained or branched alkoxycarbonyl group having 1 - 4 carbon atoms (e.g., methoxycarbonyl or ethoxycarbonyl), a phenyl-substituted straight-chained or branched alkyl group having 1 - 4 carbon atoms (e.g., benzyl or phenethyl) and a hydroxyl-substituted straight-chained or branched alkyl group having 1 - 4 carbon atoms (e.g., hydroxymethyl or 2-hydroxyethyl)); and either one of the groups represented by the following formulae (C):

### Formulae (C)

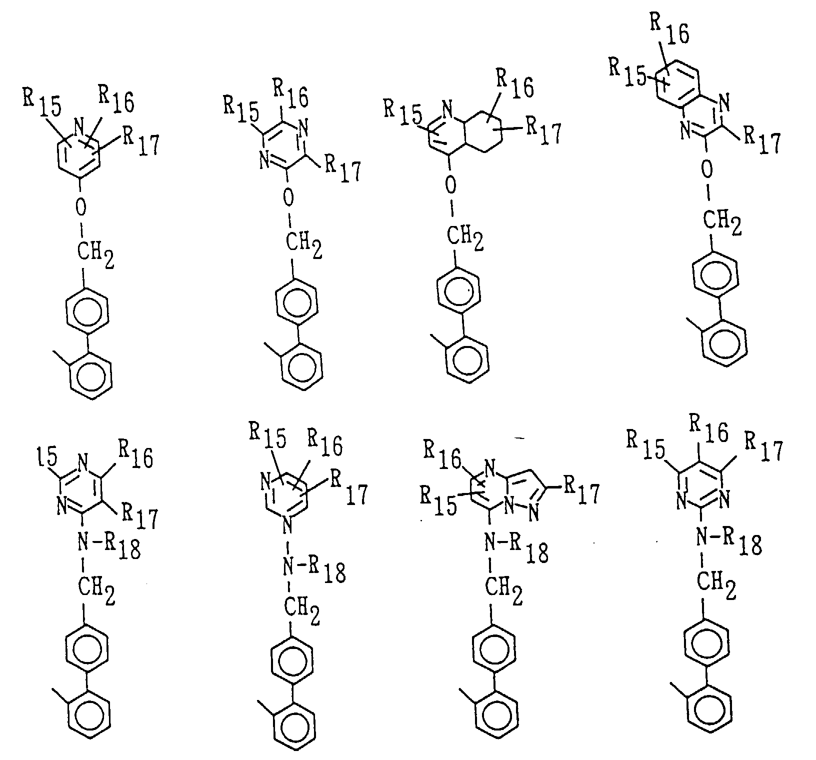

(where R₁₅, R₁₆, R₁₇ and R₁₈ are independently selected from the group consisting of hydrogen, a straight-chained or branched alkyl group having 1 - 4 carbon atoms (e.g., methyl, ethyl, n-propyl, isopropyl, n-butyl or t-butyl), a hydroxyl group, a phenyl group, a halogen atom which is either one of F, Cl, Br and I, a trifluoroacetyl group, a pentafluoropropionyl group, a straight-chained or branched alkoxycarbonyl group having 1 - 4 carbon atoms (e.g., methyoxycarbonyl or ethoxycarbonyl), a phenyl-substituted straight-chained or branched alkyl group having 1 - 4 carbon atoms (e.g., benzyl or phenethyl) and a hydroxyl-substituted straight-chained or branched alkyl group having 1 - 4 carbon atoms (e.g., hydroxymethyl or 2-hydroxyethyl)).

Specific examples of the groups represented by the formulae (A) include the following:

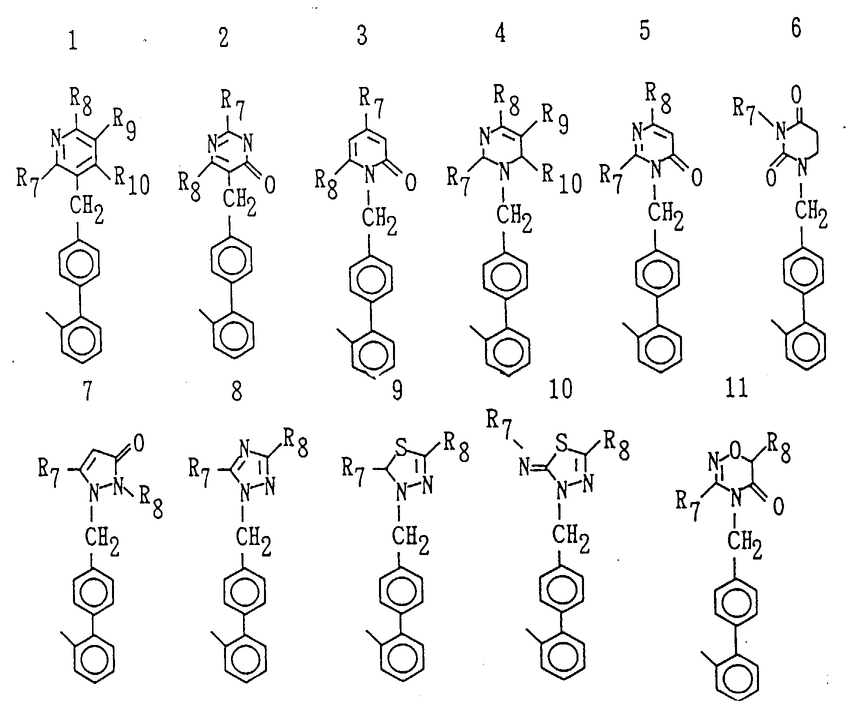

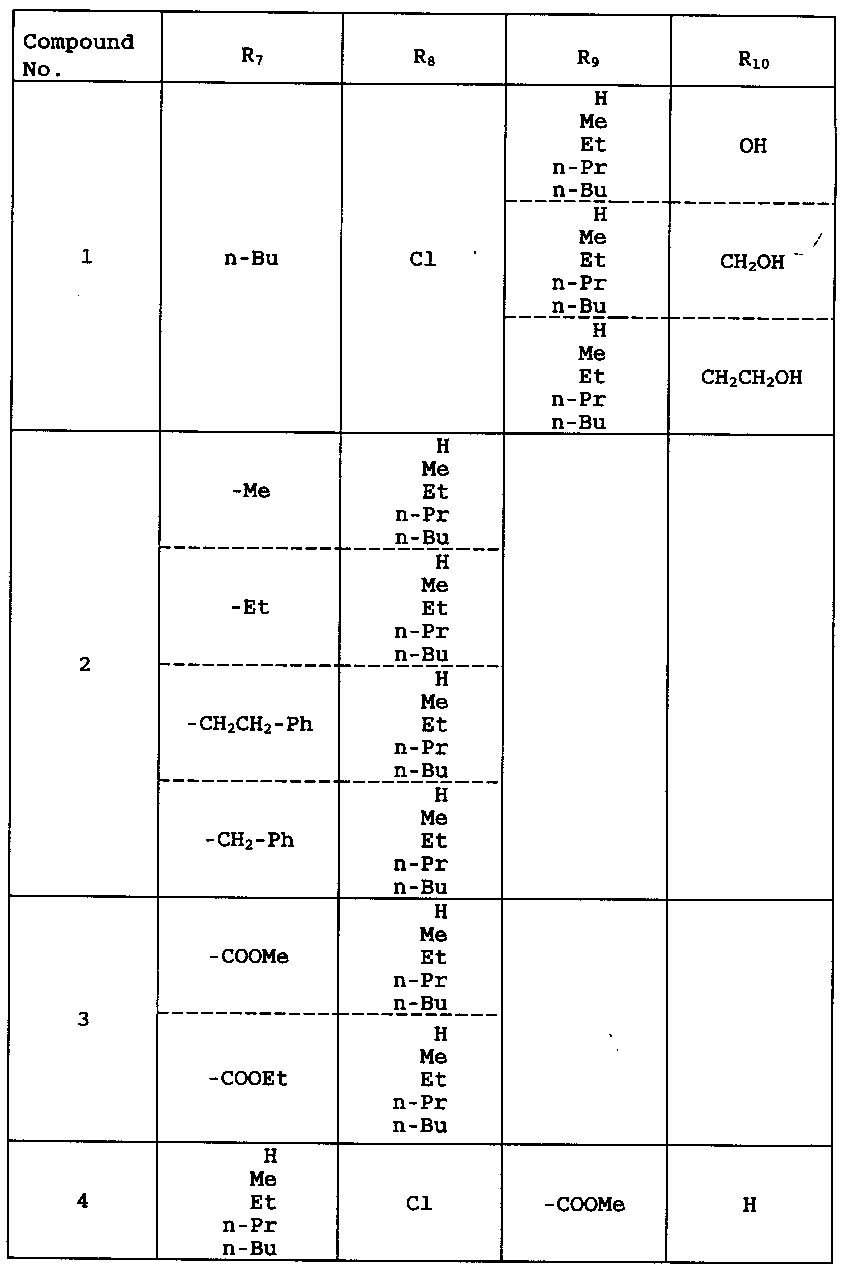

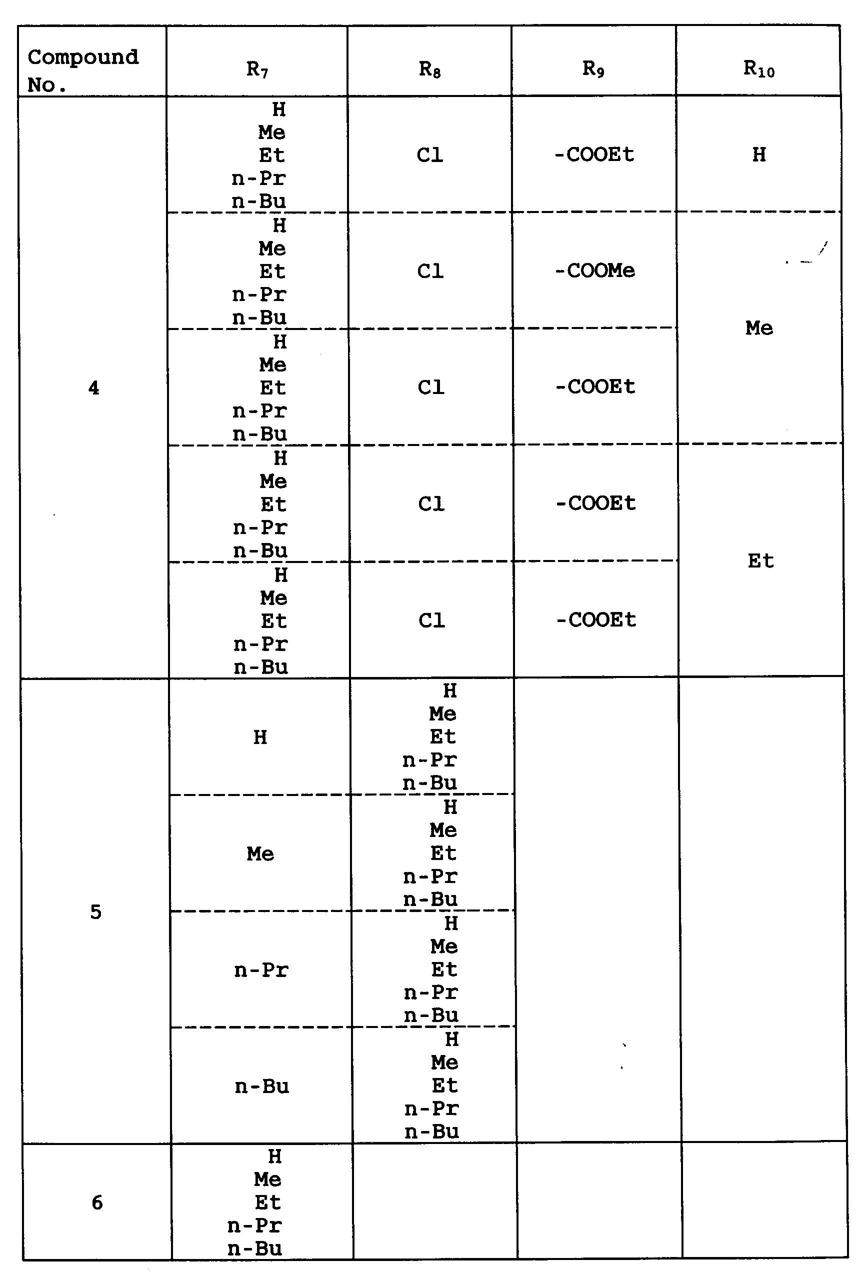

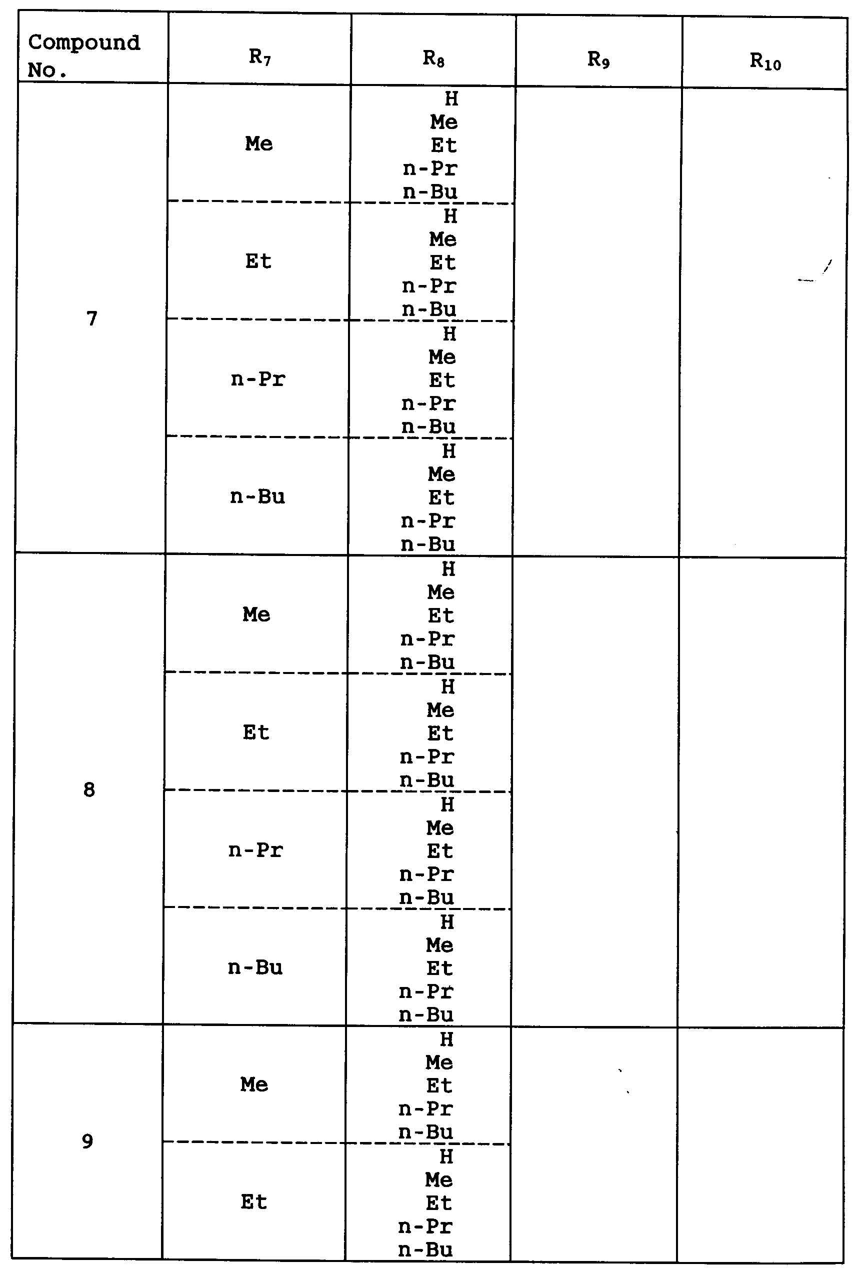

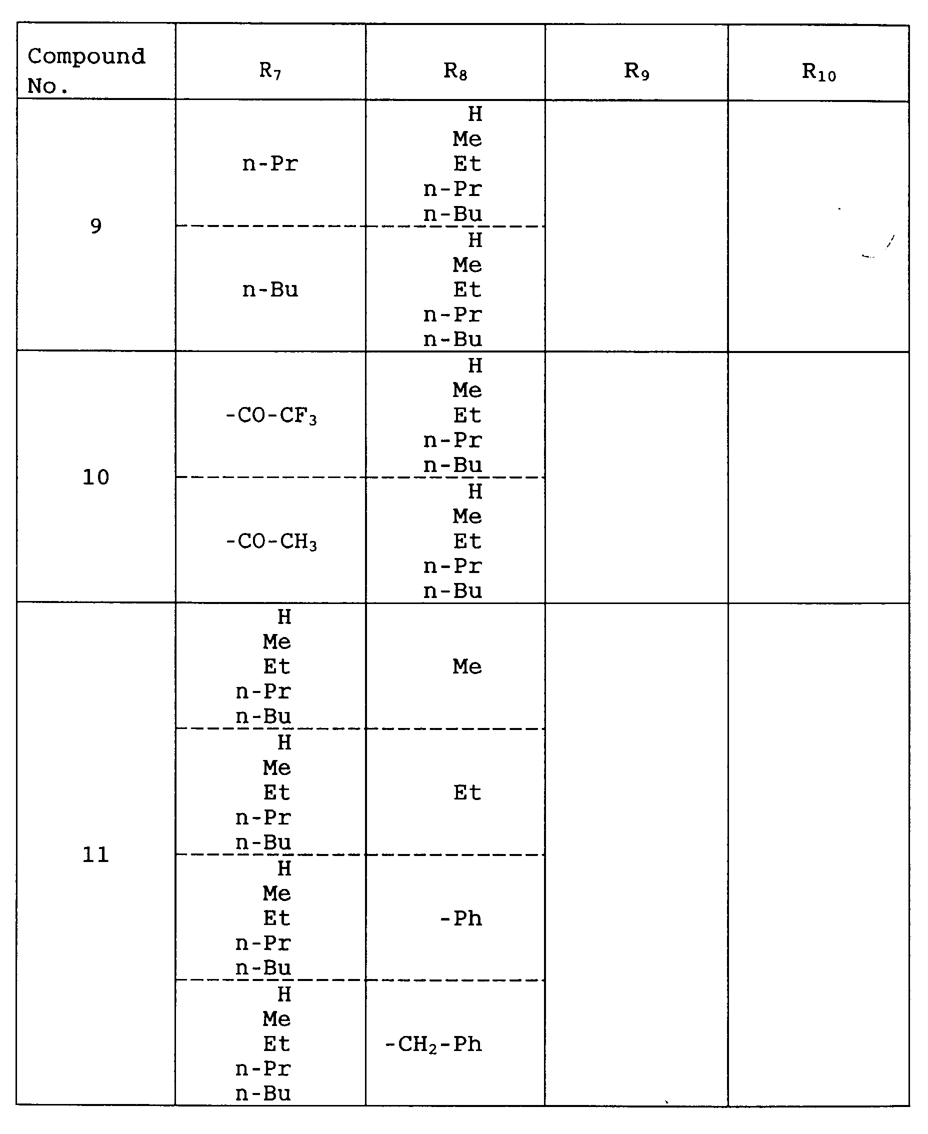

Specific examples of the groups represented by the formulae (B) include the following:

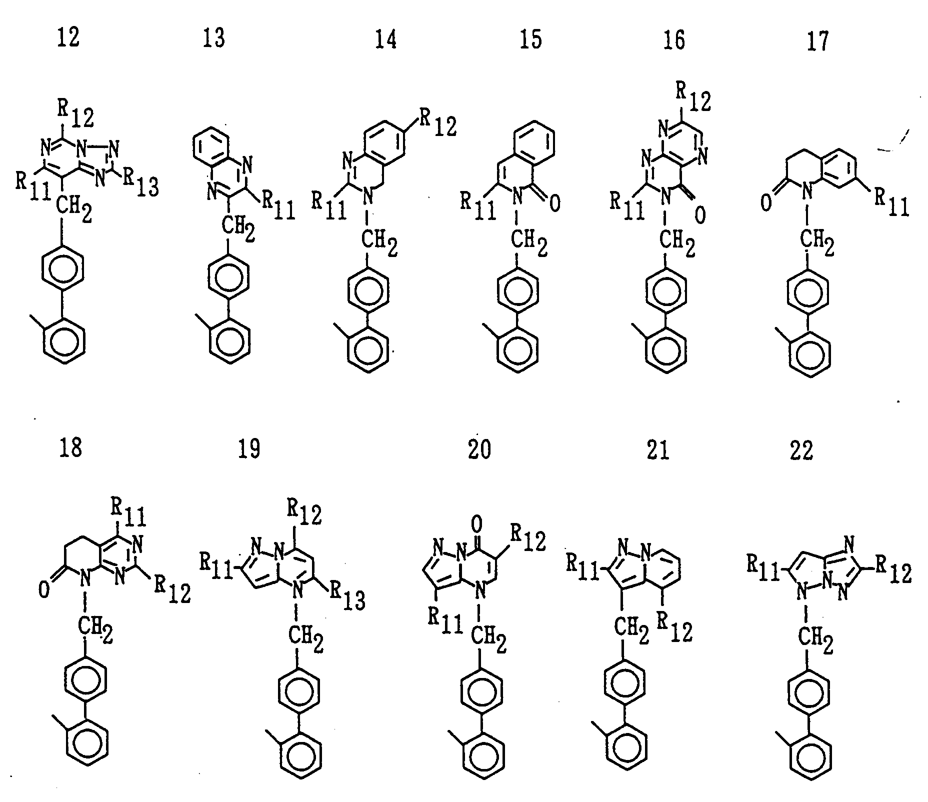

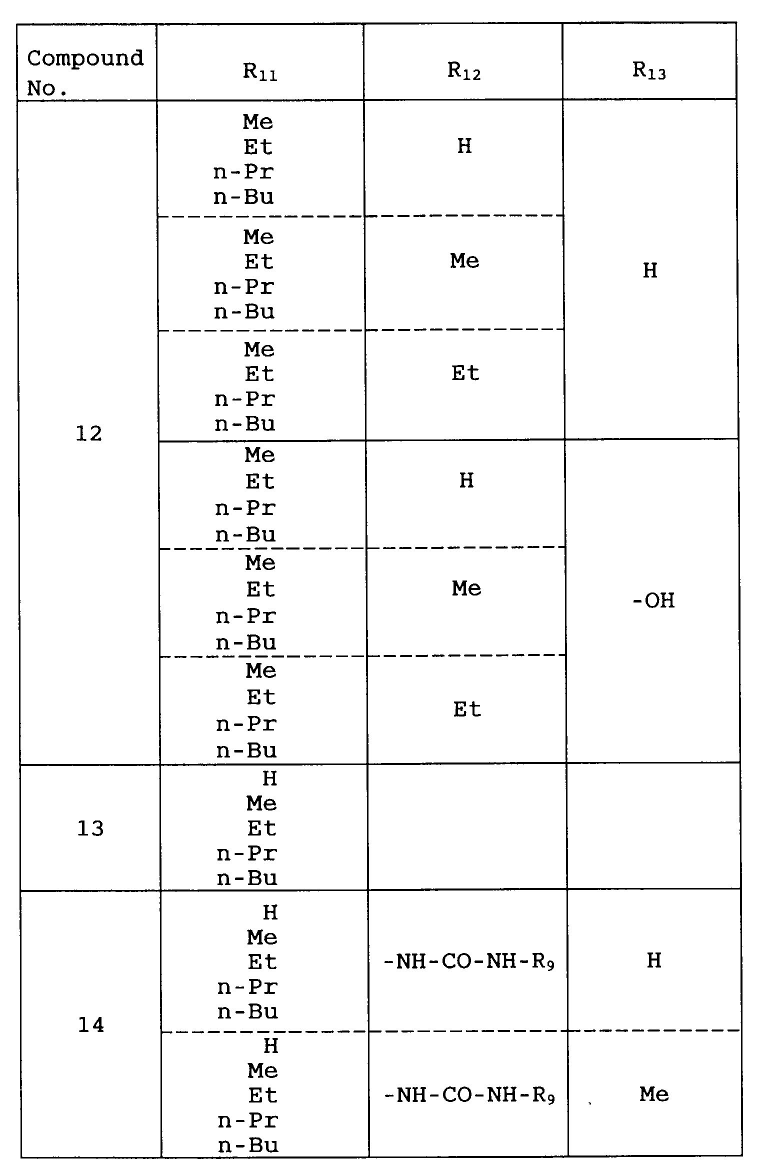

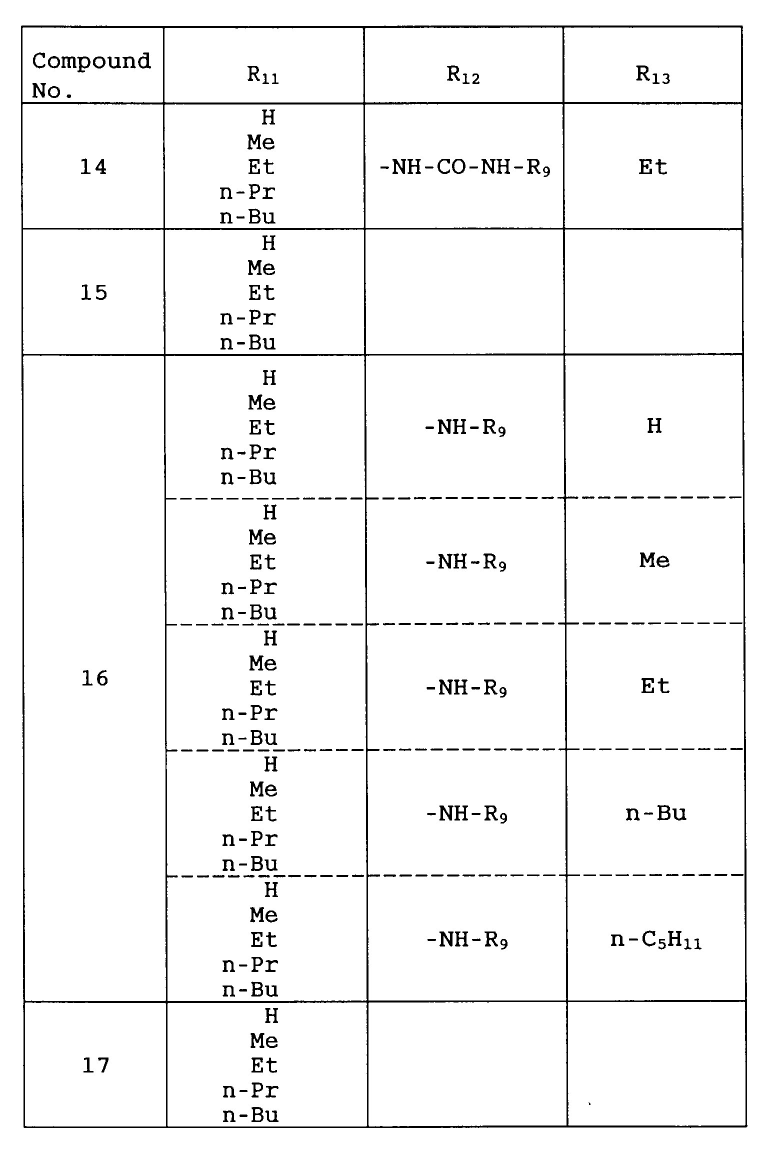

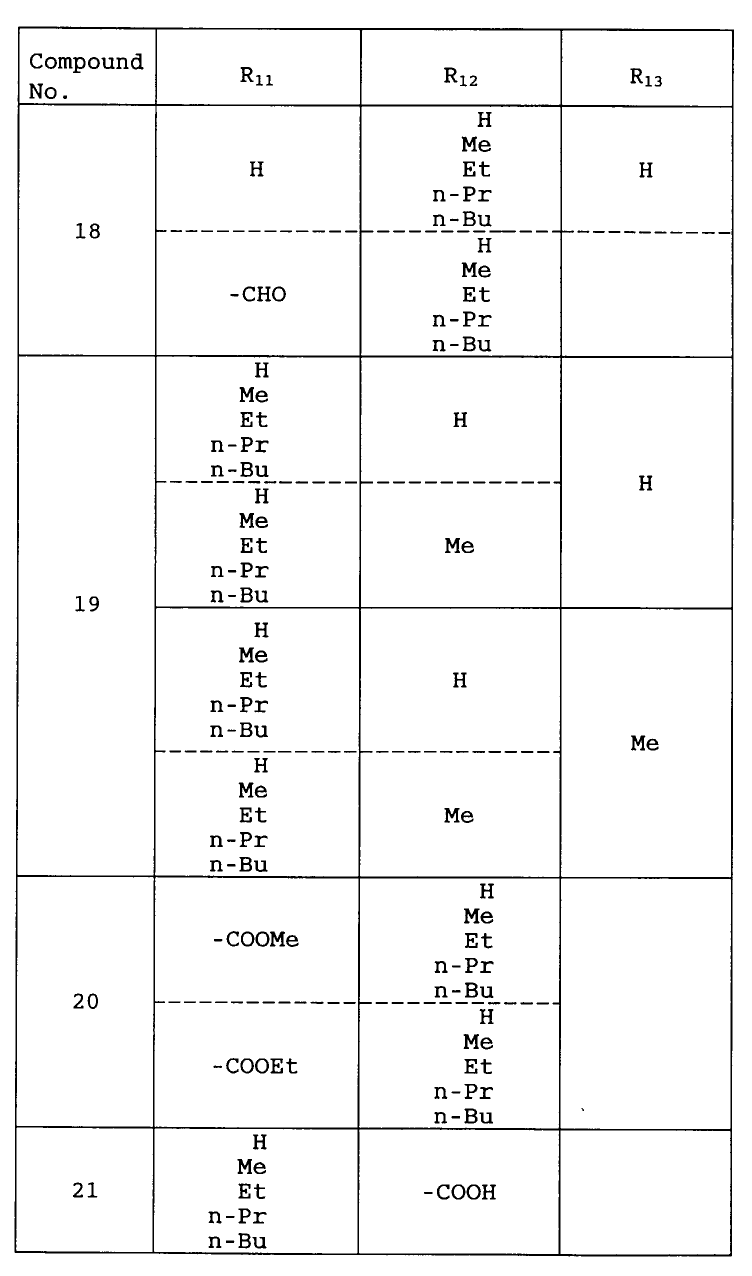

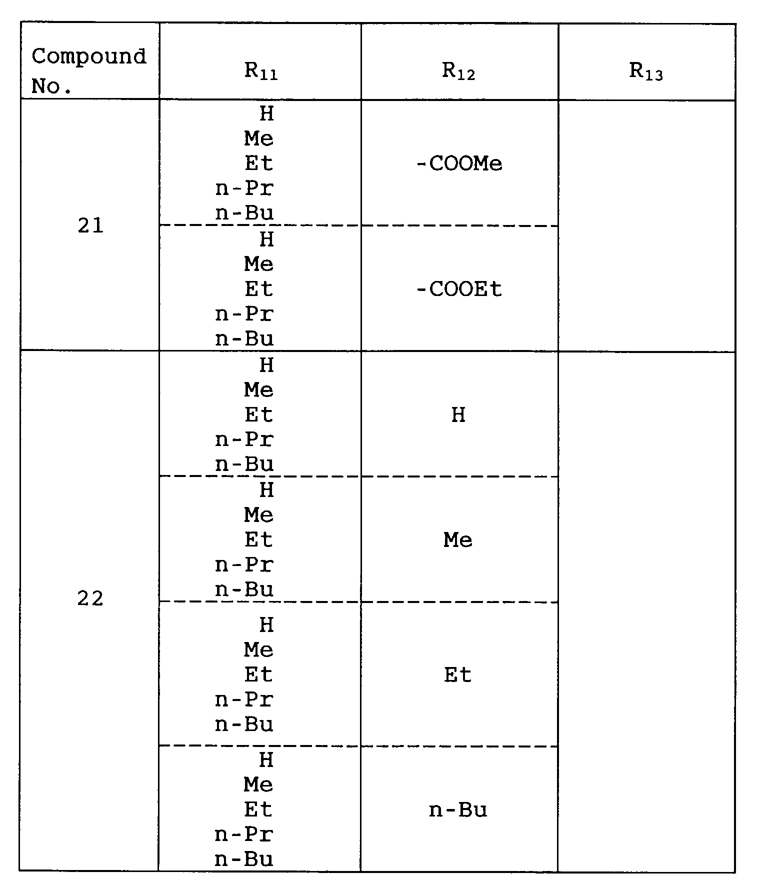

Specific examples of the groups represented by the formulae (C) include the following:

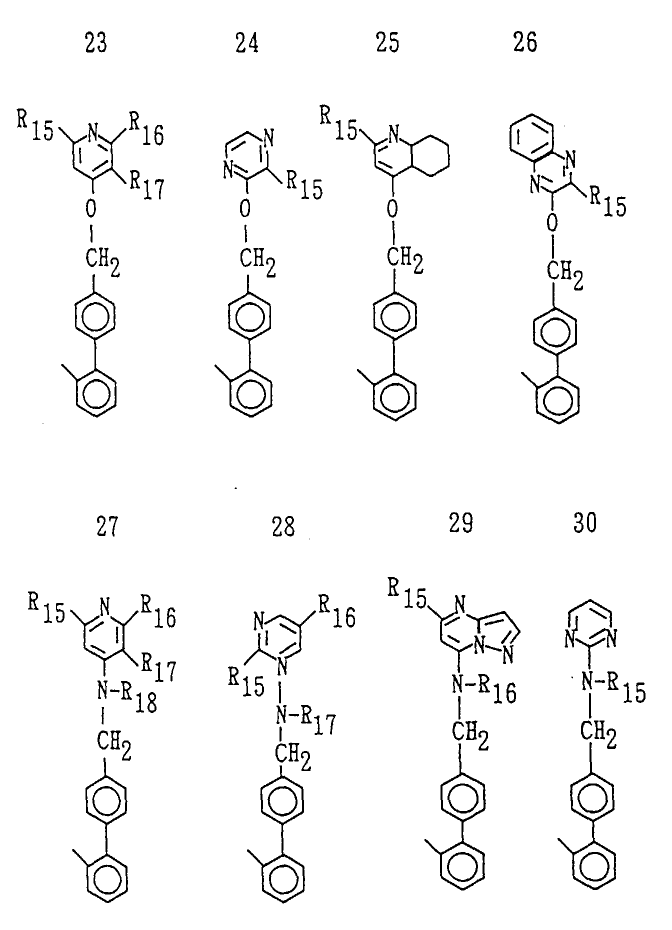

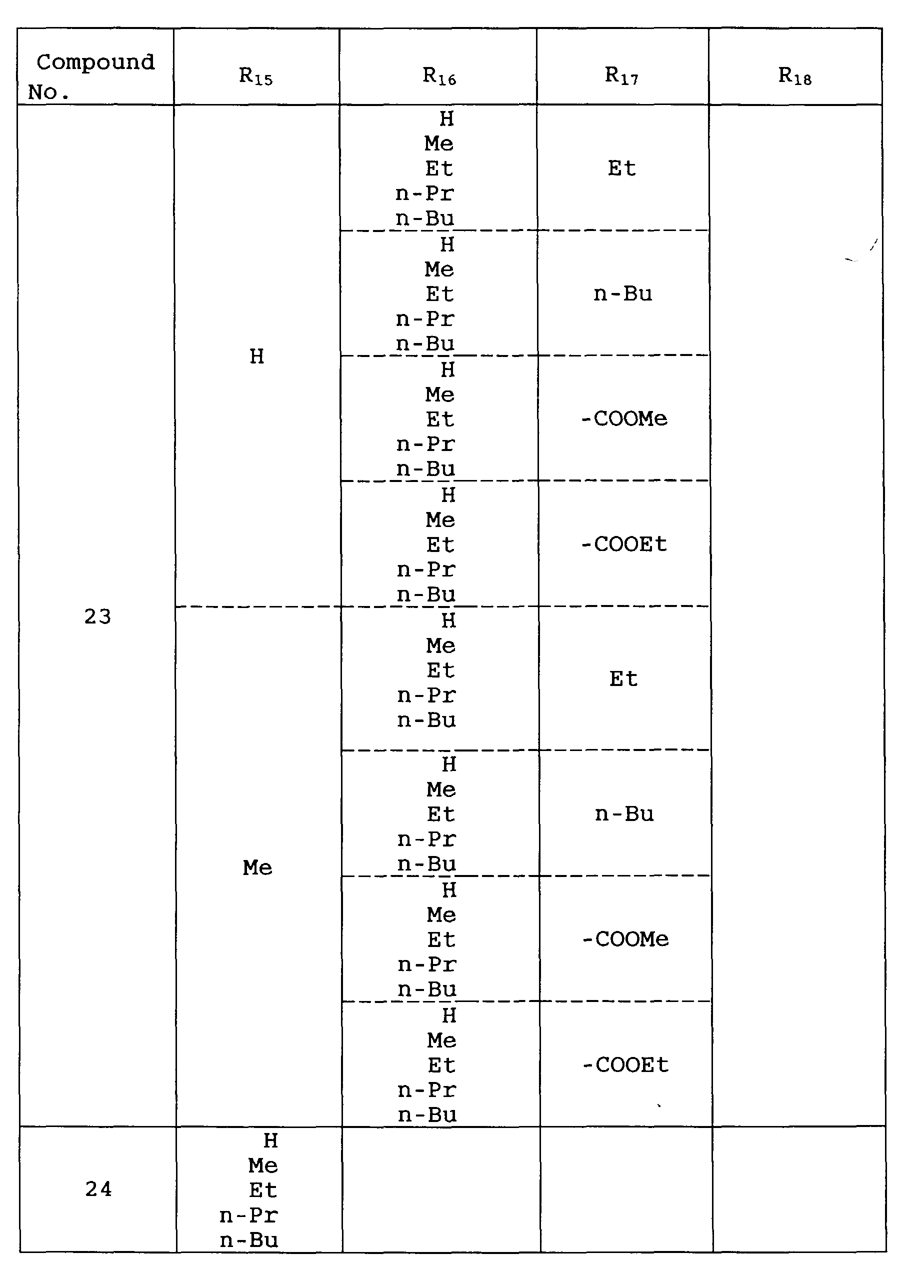

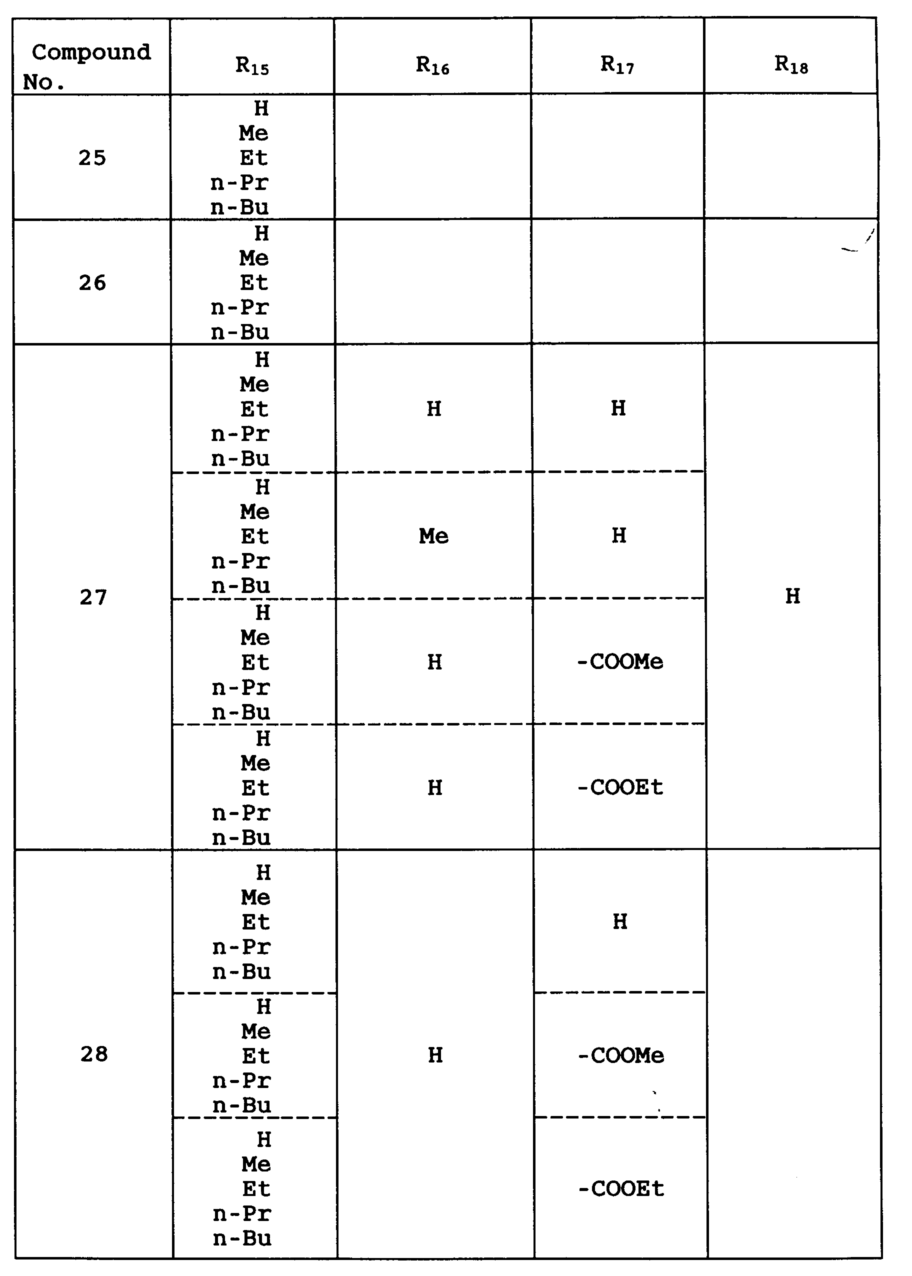

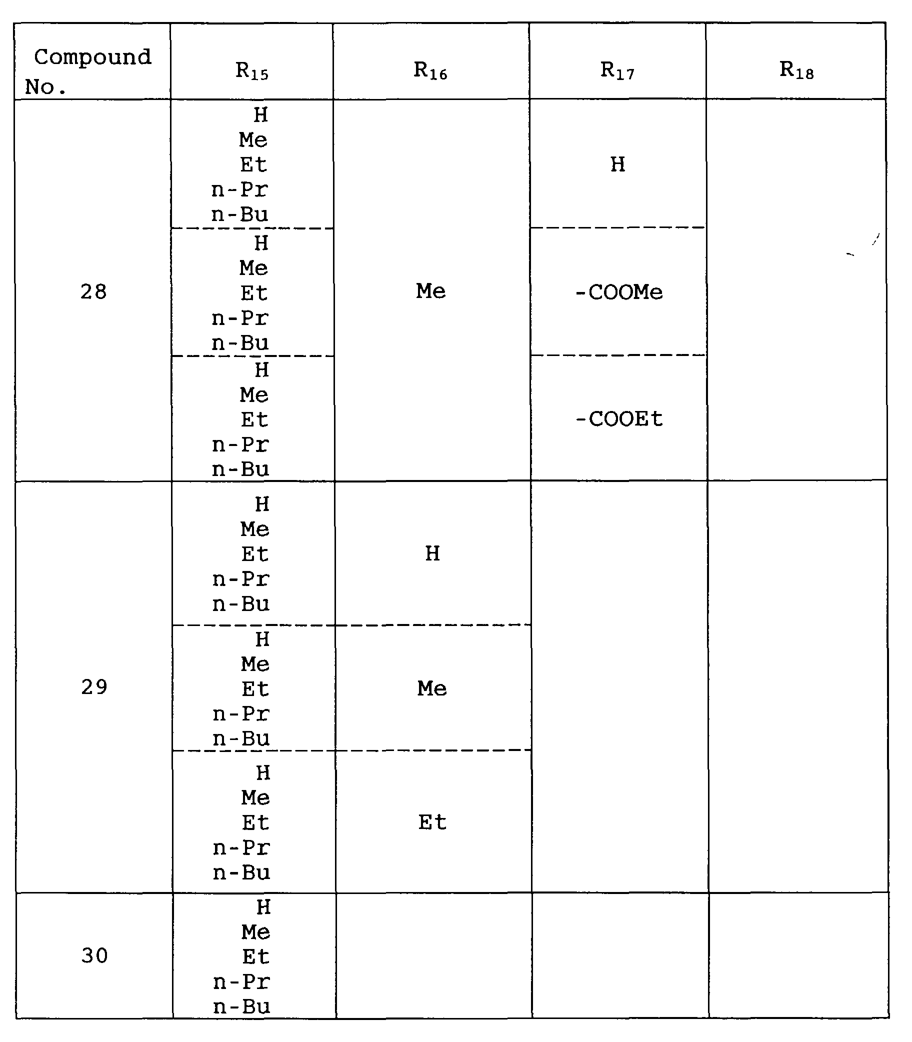

The production of 1H-tetrazole compounds using the synthesis reagent of the invention is typically performed under the conditions of atmospheric pressure at 50 - 150°C for 30 min to 24 h. If necessary, tetrazole compounds may be produced in the present invention using reaction solvents such as methyl ethyl ketone, DMF, n-butanol, amyl alcohol, n-hexyl alcohol, ethanol, isopropyl alcohol, heptyl alcohol, DMSO, chloroform, dichloroethane, THF, dioxane, N-methylpyrrolidone, 1,3-dimethylimidazolidinone and t-butyl methyl ether.

### Examples

The following examples are provided to further illustrate the present invention but are in no way to be taken as limiting.

### Example 1: Production of 5-ethyoxycarbonyl-1H-tetrazole

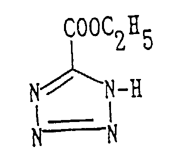

A 200-mL glass reactor fitted with a thermometer, a dropping funnel and a reflux condenser was charged with 14.4 g (105.6 mmol) of zinc chloride, 13.7 g (211.2 mmol) of sodium azide and 70 mL of methyl ethyl ketone and the contents were stirred on a water bath. To the resulting mixture, 10.15 g (102.5 mmol) of ethyl cyanoformate was added dropwise through a dropping funnel over a period of about 30 min; thereafter, the temperature was raised and reaction was performed at an internal temperature of 60 - 70°C for 1 h.

After the end of the reaction, cooling was effected and excess dilute HCl was added, followed by stirring for about 1 h. Methyl ethyl ketone was distilled off under vacuum and the residual aqueous layer was subjected to extraction (continuous extraction) with tert-butyl methyl ether; after drying with MgSO₄, the solvent was distilled off to give a white crystal in an amount of 12.8 g. Upon recrystallization with benzene, a tetrazole derivative of high purity was produced in an amount of 11.68 g (yield after recrystallization: 80.2%). m.p.: 90.4 - 91.6°C.

### Example 2: Production of 5-methyl-1H-tetrazole

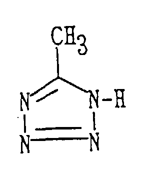

Acetonitrile (50 mL), 13 g (95.4 mmol) of zinc chloride and 12.4 g (190.7 mmol) of sodium azide were charged into a 100-mL glass reactor fitted with a thermometer and reflux condenser. With stirring, the mixture was heated under reflux to effect reaction for about 10 h.

Dilute HCl was added to the reaction mixture and after forming a uniform layer, the unreacted acetonitrile was recovered under vacuum. The residue (aqueous layer) was subjected to extraction (continuous extraction) and after drying of the ethyl acetate layer with MgSO₄, the solvent was distilled off under vacuum to give 5-methyl 1H-tetrazole as white crystal. Crude product, 7.4 g (yield, 92.5%). m.p.: 146.2-147.0°C.

### Example 3: Production of 5-(4'-methylbiphenyl-2-yl)-1H-tetrazole

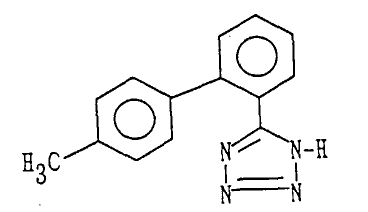

A mixture of 5 g (25.9 mmol) of 4'-methylbiphenyl-2-carbonitrile, 7.06 g (51.8 mmol) of zinc chloride, 6.73 g (103.6 mmol) of sodium azide and 15 mL of DMF was reacted at 110 - 120°C for 15 h in a 50-mL glass reactor fitted with a thermometer and a reflux condenser.

After confirming the disappearance of the starting materials by TLC, cooling was effected and dilute HCl was added slowly after the internal temperature became 100°C or below. The precipitating white crystal was recovered by filtration, washed with water and dried under vacuum to give a tetrazole derivative of high purity in an amount of 5.74 g (yield, 94.0%). m.p.: 147.5 - 148.5°C.

### Example 4: Production of 5-phenyl-1H-tetrazole

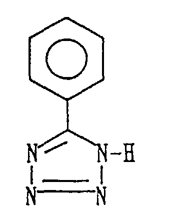

A mixture of 10 g (97.0 mmol) of benzonitrile, 14.5 g (106.7 mmol) of zinc chloride, 13.9 g (213.3 mmol) of sodium azide and 20 mL of DMF was charged into a 100-mL glass reactor fitted with a thermometer and a reflux condenser and under stirring, the mixture was reacted at an internal temperature of 100 - 110°C for 30 min.

After confirming the disappearance of the starting materials by TLC, cooling was effected and excess dilute HCl was added to render the mixture acidic, followed by stirring for several hours with the mixture being held in the acidic state. The precipitating crystal was recovered by filtration, washed with water and dried under vacuum to give 5-phenyl-1H-tetrazole of high purity in an amount of 13.7 g (yield: 96.6%). m.p.: 215.5 - 217.0°C.

### Example 5: Production of 5-(2-bromophenyl)-1H-tetrazole

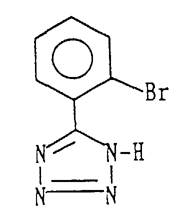

A mixture of 8.83 g (48.5 mmol) of 2-bromobenzonitrile, 7.27 g (53.4 mmol) of zinc chloride, 6.94 g (106.8 mmol) of sodium azide and 10 mL of DMF was charged into a 50-mL glass reactor fitted with a thermometer and a reflux condenser, and under stirring, the mixture was reacted at an internal temperature of 100 - 110°C for 2 h.

After confirming the disappearance of the starting materials by TLC, post-treatments were performed as in Example 4. A high-purity white crystal of 5-(2-bromophenyl)-1H-tetrazole was obtained in an amount of 9.44 g (yield, 86.5%). m.p.: 181.5 - 182.5°C.

### Example 6: Production of 5-ethyl-2-imino-3-[2'-(1H-tetrazol-5-yl)-biphenyl-4-yl]methyl-1,3,4-thiadiazoline

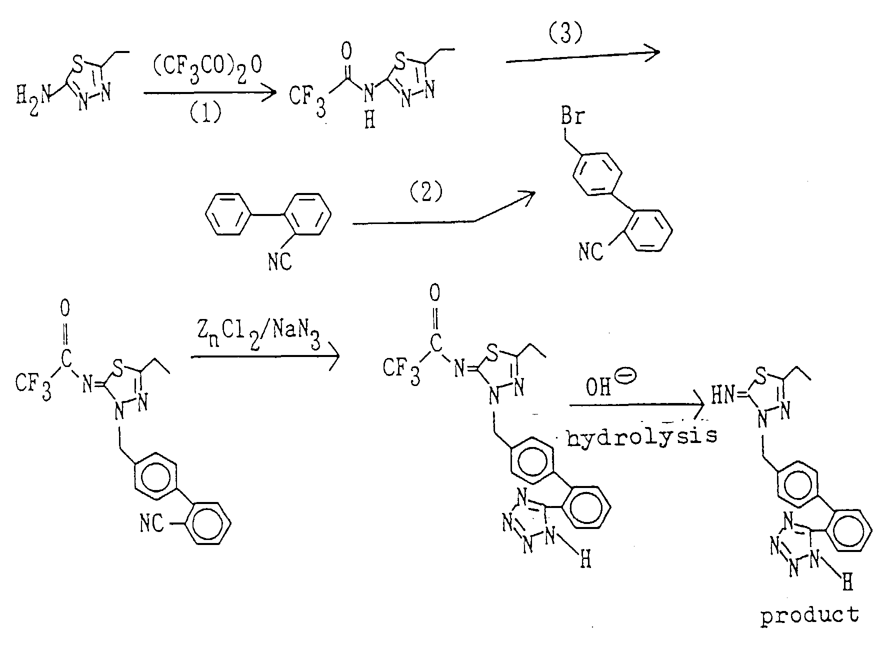

The end compound was produced by the following four steps (1) - (4).

### (1) Production of 2-trifluoroacetamido-5-ethyl-1,3,4-thiadiazole

To a suspension of 200 g of 2-amino-5-ethyl-1,3,4-thiadiazole in 3 L of toluene was added to 260 mL of triethylamine at room temperature; under cooling with ice, to the mixture was added dropwise 265 mL of trifluoroacetic anhydride, and the mixture was stirred at room temperature for 1 h. Water was added to the reaction mixture, and the crystalline precipitate was recovered by filtration. Further, ethyl acetate was added to the filtrate and the organic layer was separated off and dried with anhydrous magnesium sulfate; thereafter, the solvent was distilled off under vacuum to yield the title compound in an amount of 237.5 g (68%).

### (2) Production of 4-bromomethyl-2'-cyanobiphenyl

4-Methyl-2'-cyanobiphenyl (10.5 g), N-bromosuccinimide (9.79 g) and 2,2'-azodiisobutyronitrile (120 mg) were added to 110 mg of carbon tetrachloride and the mixture was heated under reflux for 2 h. The insolubles were removed by filtration while hot and the filtrate was left to cool; thereafter, the crystalline precipitate was recovered by filtration to give the title compound in an amount of 6.6 g (44%).

### (3) Production of 5-ethyl-2-trifluoroacetylimino-3-(2'-cyanobiphenyl-4-yl)methyl-1,3,4-thiadiazoline

Sodium hydride (124.1 g; dispersion in 55% oil) was suspended in 1.5 L of N,N-dimethylformamide, and to the ice-cooled suspension was added 533.7 g of 2-trifluoroacetamido-5-ethyl-1,3,4-thiadiazole prepared in (1) above on ice. After the evolution of hydrogen gas ceased, a solution of 4-bromomethyl-2'-cyanobiphenyl (643.6 g) obtained in (2) above in 3L of N,N-dimethylformamide was added dropwise to the mixture. The mixture was stirred at room temperature for 1 h, then, stirred at 80°C for 5 h. The reaction mixture was concentrated under vacuum; after the addition of water and ethyl acetate, the organic layer was washed with water, dried over anhydrous magnesium sulfate and had the solvent distilled off under vacuum. The residue was subjected to column chromatography on silica gel and eluted with a 3:1 mixture of hexane and ethyl acetate. The crude crystal was recrystallized from ethanol to give the title compound in an amount of 490.8 g (50%).

### (4) Production of 5-ethyl-2-imino-3-[2'-(1H-tetrazol-5-yl)biphenyl-4-yl]methyl-1,3,4-thiadiazoline

A 100-mL glass reactor fitted with a thermometer, a reflux condenser, a calcium chloride tube and a stirrer was charged with 30 mL of n-BuOH, 6.0 g (44 mmol) of zinc chloride and 7.4 g (113.8 mmol) of sodium azide and, the contents were stirred for 30 min. To the mixture was charged 9.5 g (22.8 mmol) of the 5-ethyl-2-trifluoroacetylimino-3-(2'-cyanobiphenyl-4-yl)methyl-1,3,4-thiadiazoline obtained in (3) above, and the mixture was heated to an internal temperature of 105 - 115°C, at which temperature reaction was performed for 24 h.

After confirming the disappearance of the starting materials by TLC, chloroform and water were added in appropriate amounts; following the treatment with 10% H₂SO₄, and the organic layer was separated. To the organic layer was added a 5% aqueous NaOH solution, and the mixture was stirred for several hours; after confirming the completion of hydrolysis by TLC, the aqueous layer was separated. The recovered alkaline aqueous layer was adjusted to pH of 5.5 - 6.0 with 3 N aqueous HCl solution, whereupon a white crystal came out of the solution; the crystal was separated by filtration and dried to give the desired compound in an amount of 6.5 g (crude yield, 78.5%).

### Example 7: Production of 5-ethyl-2-imino-3-[2'-(1H-tetrazol-5-yl)-biphenyl-4-yl]methyl-1,3.4-thiadiazoline

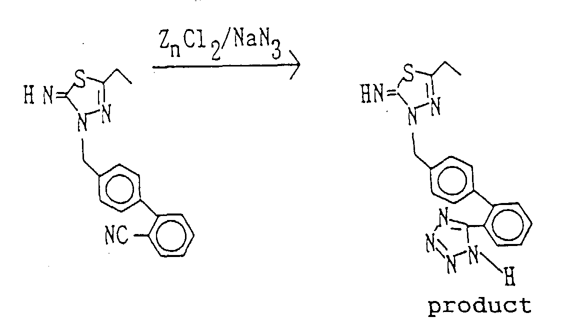

A 1000-mL glass reactor fitted with a thermometer, a reflux condenser and a stirrer was charged with 300 mL of n-BuOH, 73.9 g (0.54 mmol) of zinc chloride and 71.0 g (1.1 mol) of sodium azide and the mixture was stirred for 30 min. To the mixture was additionally charged 90.0 g (0.28 mol) of 5-ethyl-2-imino-3-(2'-cyanobiphenyl-4-yl)methyl-1,3,4-thiadiazoline and the mixture was heated to an internal temperature of 105 - 115°C, at which temperature reaction was performed for 24 h.

After confirming the disappearance of the starting materials by TLC, chloroform and water were added in appropriate amounts; following the treatment with 10% H₂SO₄, and the organic layer was separated. To the organic layer, there was added a 5% aqueous sodium hydroxide solution, and the mixture was stirred for several hours; thereafter, the aqueous layer was separated. The recovered alkaline aqueous layer was adjusted to pH of 5.5 - 6.0 with 3 N aqueous HCl solution, whereupon a white crystal came out of solution. The crystal was separated by filtration and dried to give the desired compound in an amount of 76.5 g (crude yield, 75%).

### Industrial Applicability

By using the tetrazole agent of the invention, the following advantages are anticipated, therefore, the present invention is extremely useful as an industrial process for the production of 1H-tetrazole compounds.
(1) Instead of the expensive trialkyltin azides used in the method (B) or (C), the present invention employs inexpensive zinc chloride to thereby reduce the materials cost. In addition, tin compounds which are highly toxic (to cause skin eruptions) can be dispensed with.
(2) Compared with the method (D), the invention process permits many kinds of solvents to be used and may be described as having high flexibility in use. In addition, zinc chloride is less expensive than aluminum chloride (the price of the former per kilogram is about 60% of the latter); what is more, compared with aluminum chloride which requires three molar equivalents of an alkali metal azide, zinc chloride requires only two molar equivalents of the same, thus leading to cost reduction.
(3) The applicability of the zinc chloride/alkali metal azide which is used in the invention is by no means limited to nitrile forms having steric hindrance and they can in principle be used with any nitrile forms.

## Claims

1. A reagent for the synthesis of 1H-tetrazoles of the formula (II): 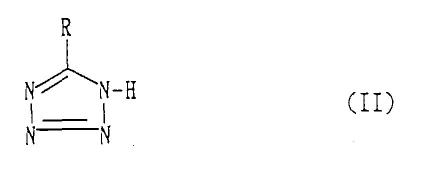 (where R is any substituent) from carbonitriles of the formula (I):
R - CN (I)
(where R has the same meaning as defined above), said reagent being characterized by comprising an alkali metal azide and zinc chloride.

2. The reagent for synthesis according to claim 1, wherein the alkali metal azide is sodium azide.

3. The reagent for synthesis according to claim 1 or 2, wherein R is selected from the group consisting of hydrogen, an optionally substituted alkyl group, an optionally substituted alkenyl group, an optionally substituted alkynyl group, an optionally substituted alkoxy group, an optionally substituted alkylthio group, an optionally substituted alkylsulofnyl group, an optionally substituted alkylsulfinyl group, an optionally substituted alkylamino group, an optionally substituted alkenyloxy group, an optionally substituted alkoxycarbonyl group, an optionally substituted aryl group, an optionally substituted alryloxy group, an optionlly substituted arylthio group, an optionally substituted aralkyl group, an optionally substituted arylalkenyl group and optionally substituted heterocyclic group.

4. The regent for synthesis according to claim 1 or 2, wherein R is selected from the group consisting of: hydrogen; a straight-chained or branched, optionally substituted alkyl group having 1 - 4 carbon atoms; a straight-chained or branched, optionally substituted alkoxy group having 1 - 4 carbon atoms; a straight-chained or branched, optionally substituted alkylsulfonyl group having 1 - 4 carbon atoms; a phenyl group; a monosubstituted phenyl group of the following formula: 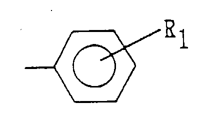 (where R₁ is selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a disubstituted phenyl group of the following formula: 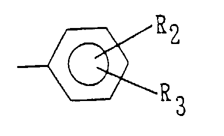 (where R₂ and R₃ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a 4'-methylbiphenyl-2-yl group; a group of the formula: 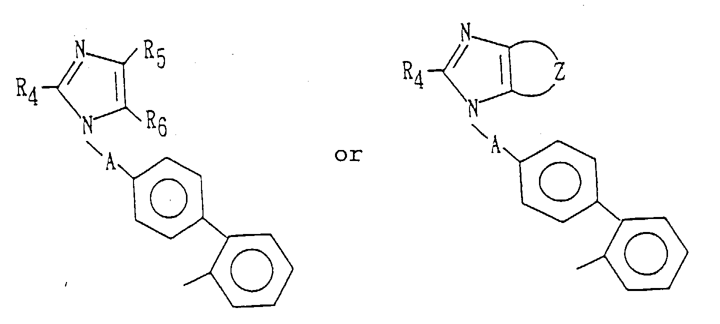 (where A is either O, NH or CH₂; Z is a divalent group selected as having either one of the following formulae: 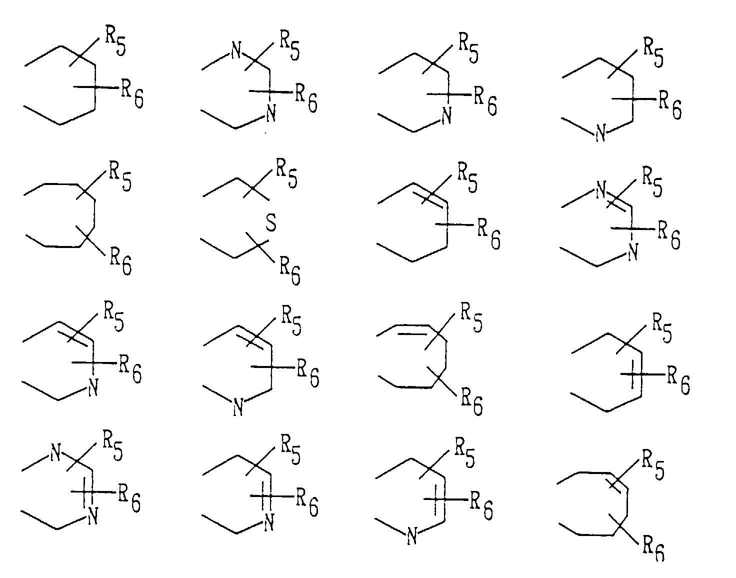 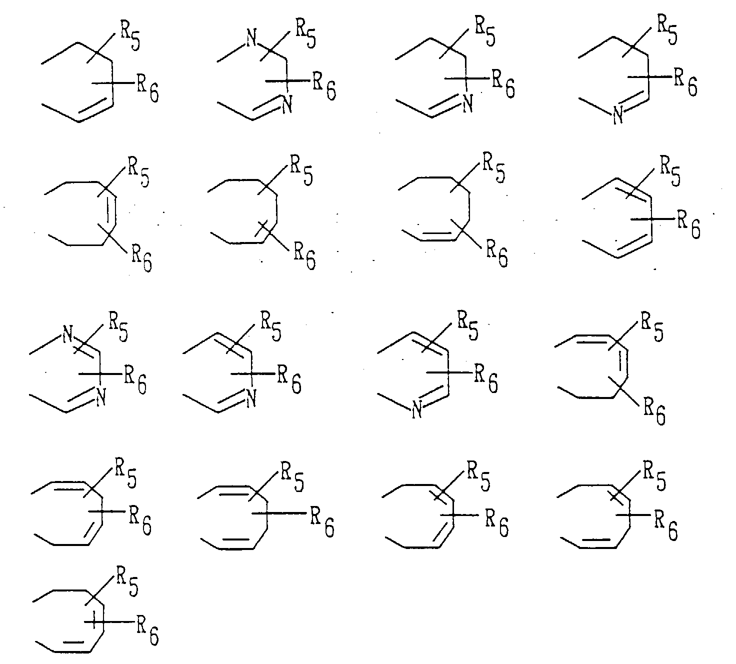 R₄, R₅ and R₆ are independently selected from the group consisting of a halogen atom which is either one of F, Cl, Br and I, a straight-chained or branched alkyl group having 1 - 4 carbon atoms, a straight-chained or branched alkoxy group having 1 -4 carbon atoms, a straight-chained or branched alkylsulfonyl group having 1 - 4 carbon atoms, a nitro group, an amino group and a carboxyl group); a group of the following formula: 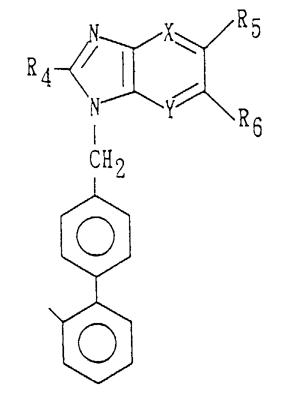 (where X and Y are independently selected from among CH and N, and R₄, R₅ and R₆ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); either one of the groups represented by the following formulae: 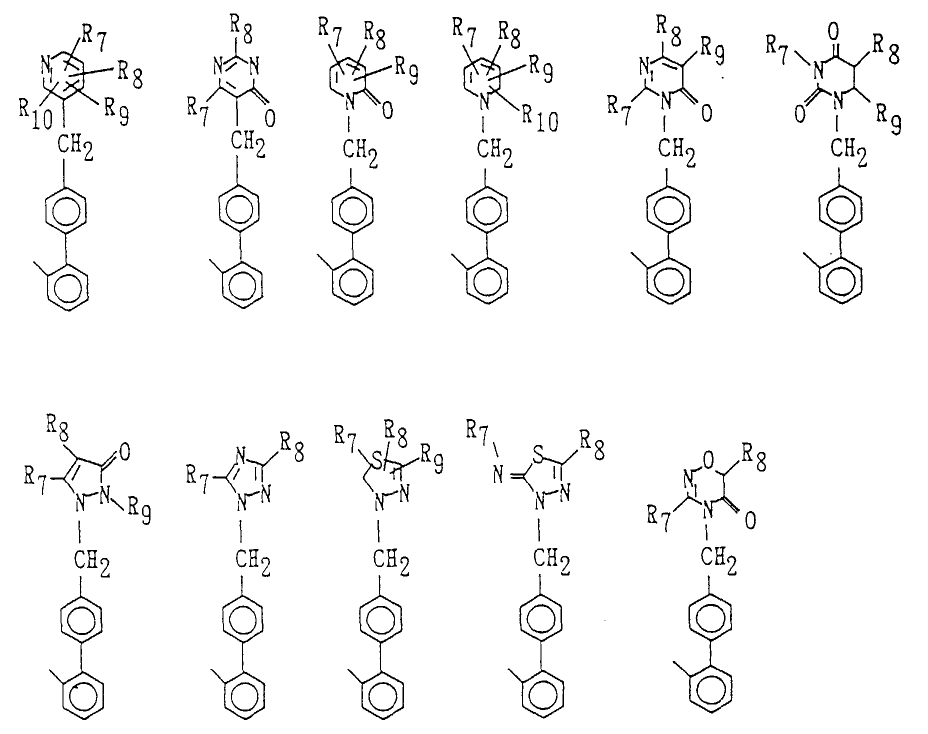 (where R₇, R₈, R₉ and R₁₀ are independently selected from the group consisting of hydrogen, a straight-chained or branched alkyl group having 1 - 4 carbon atoms, a hydroxyl group, a phenyl group, a halogen atoms which is either one of F, Cl, Br and I, a trifluoroacetyl group, a pentafluoropropionyl group, a straight-chained or branched alkoxycarbonyl group, a phenyl-substituted straight-chained or branched alkyl group having 1 - 4 carbon atoms, and a hydroxyl-substituted straight-chained or branched alkyl group); either one of the groups represented by the following formulae: 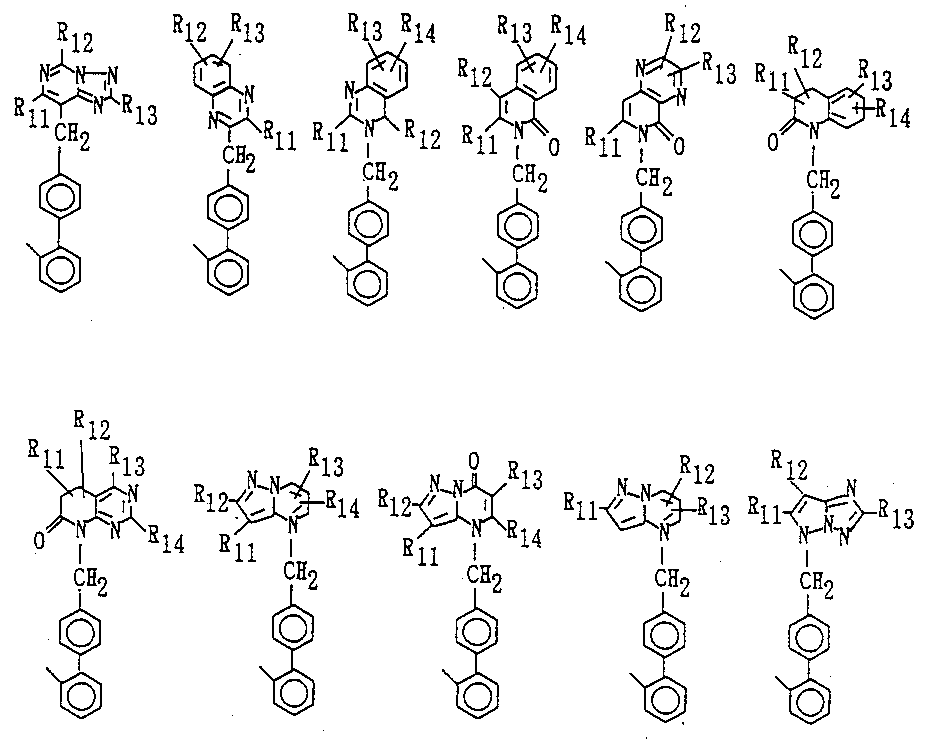 (where R₁₁, R₁₂, R₁₃ and R₁₄ are independently selected from the group consisting of hydrogen, a straight-chained or branched alkyl group having 1 - 4 carbon atoms, a hydroxyl group, a phenyl group, a halogen atom which is either one of F, Cl, Br and I, a trifluoroacetyl group, a pentafluoropropionyl group, a straight-chained or branched alkoxycarbonyl group having 1 - 4 carbon atoms, a phenyl-substituted straight-chained or branched alkyl group having 1 - 4 carbon atoms, and a hydroxyl-substituted straight-chained or branched alkyl group having 1 - 4 carbon atoms); and either one of the groups represented by the following formulae: 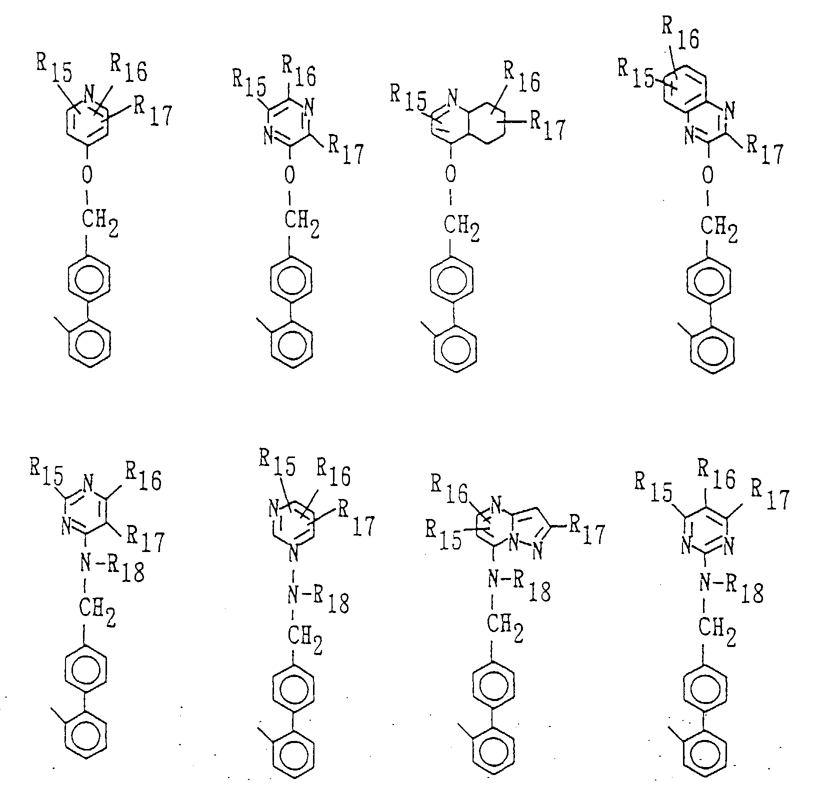 (where R₁₅, R₁₆, R₁₇ and R₁₈ are independently selected from the group consisting of hydrogen, a straight-chained or branched alkyl group having 1 - 4 carbon atoms, a hydroxyl group, a phenyl group, a halogen atom which is either one of F, Cl, Br and I, a trifluoroacetyl group, a pentafluoropropionyl group, a straight-chained or branched alkoxycarbonyl group having 1 - 4 carbon atoms, a phenyl-substituted straight-chained or branched alkyl group having 1 - 4 carbon atoms, and a hydroxyl-substituted straight-chained or branched alkyl group having 1 - 4 carbon atoms).

5. A process for producing 1H-tetrazole compounds of the formula (II): 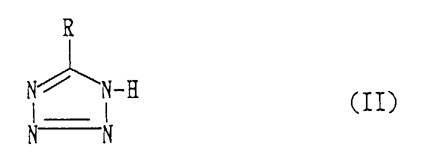 (where R is any substituent), characterized by reacting carbonitrile compounds of the formula (I):
R - CN (I)
(where R has the same meaning as defined above) with an alkali metal azide in the presence of catalytic zinc chloride.

6. The process according to claim 5, wherein the alkali metal azide is sodium azide.

7. The process according to claim 5 or 6, wherein R is selected from the group consisting of hydrogen, an optionally substituted alkyl group, an optionally substituted alkenyl group, an optionally substituted alkynyl group, an optionally substituted alkoxy group, an optionally substituted alkylthio group, an optionally substituted alkylsulfonyl group, an optionally substituted alkylsulfinyl group, and optionlly substituted alkylamino group; an optionally substituted alkenyloxy group, an optionally substituted alkoxycarbonyl group, an optionally substituted aryl group, an optionally substituted aryloxy group, an optionally substituted arylthio group, an optionally substituted aralkyl group, an optionally substituted arylalkenyl group and an optionally substituted heterocyclic group.

8. The process according to claim 5 or 6, wherein R is selected from the group consisting of: hydrogen; a straight-chained or branched, optionally substituted alkyl group having 1 - 4 carbon atoms; a straight-chained or branched, optionally substituted alkoxy group having 1 - 4 carbon atoms; a straight-chained or branched, optionally substituted alkylsulfonyl group having 1 - 4 carbon atoms; a phenyl group; a monosubstituted phenyl group of the following formula: 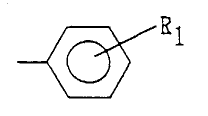 (where R₁ is selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a disubstituted phenyl group of the following formula: 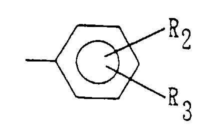 (where R₂ and R₃ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a 4'-methylbiphenyl-2-yl group; a group of the formula: 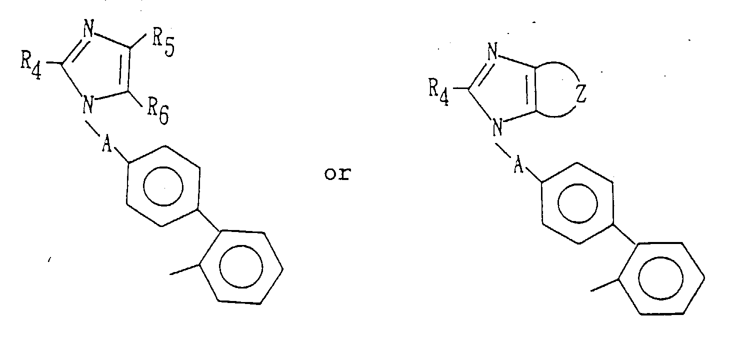 (where A is either O, NH or CH₂; Z is a divalent group selected as having either one of the following formulae: 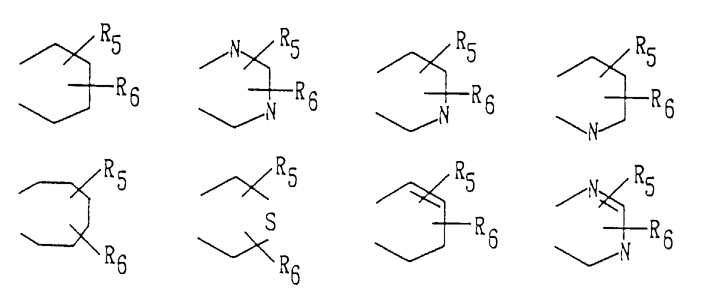 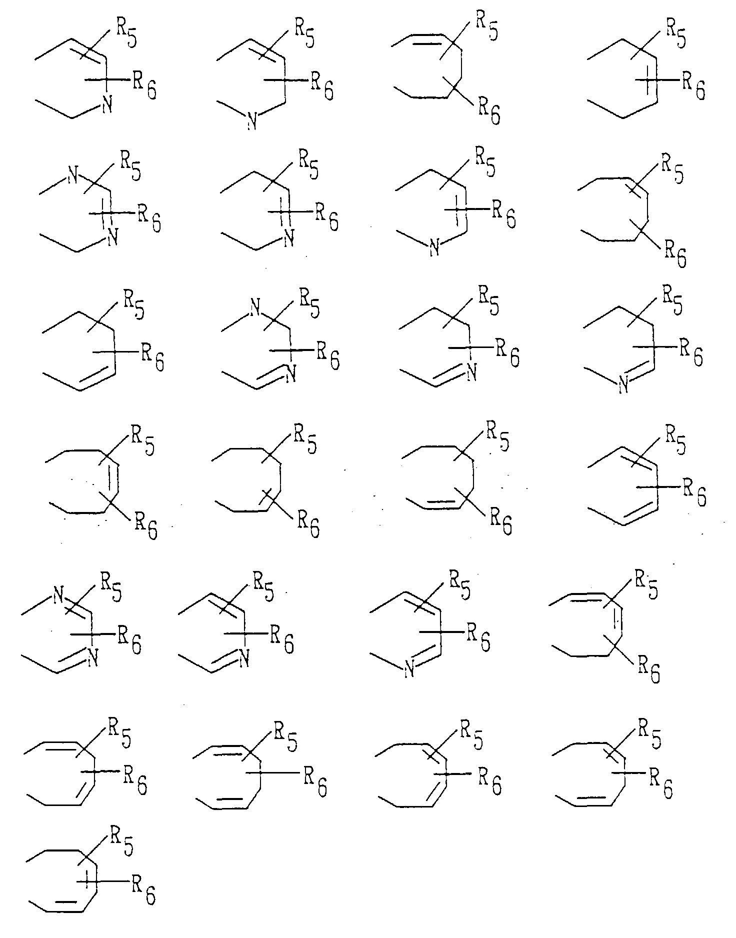 R₄, R₅ and R₆ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); a group of the following formula: 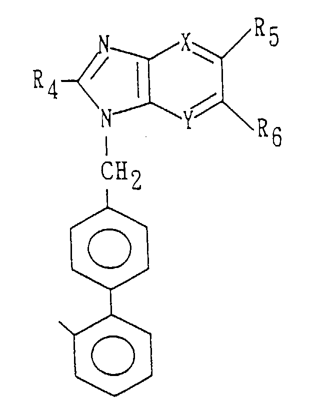 (where X and Y are independently selected from among CH and N, and R₄, R₅ and R₆ are independently selected from the group consisting of a halogen atom which is either one of F, Cl, Br and I, a straight-chained or branched alkyl group having 1 - 4 carbon atoms, a straight-chained or branched alkoxy group having 1 - 4 carbon atoms, a straight-chained or branched alkylsulfonyl group having 1 - 4 carbon atoms, a nitro group, an amino group and a carboxyl group); either one of the groups represented by the following formulae: 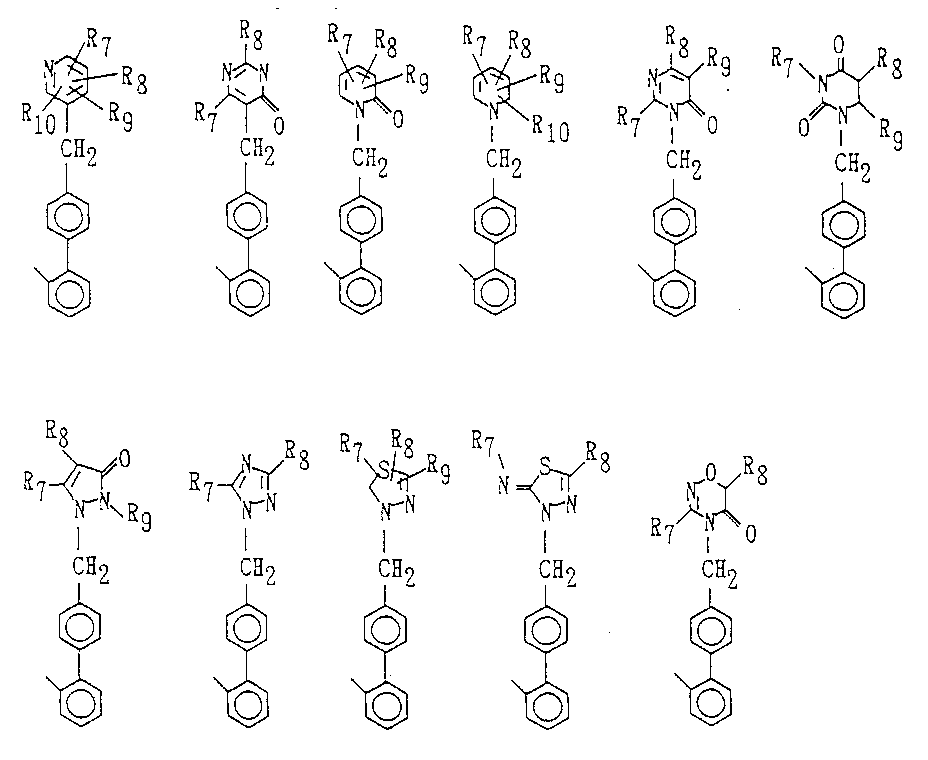 (where R₇, R₈, R₉ and R₁₀ are independently selected from the group consisting of hydrogen, a straight-chained or branched alkyl group having 1 - 4 carbon atoms, a hydroxyl group, a phenyl group, a halogen atom which is either one of F, Cl, Br and I, a trifluoroacetyl group, a pentafluoropropionyl group, a straight-chained or branched alkoxycarbonyl group, a phenyl-substituted straight-chained or branched alkyl group having 1 - 4 carbon atoms, and a hydroxyl-substituted straight-chained or branched alkyl group); either one of the groups represented by the following formulae: 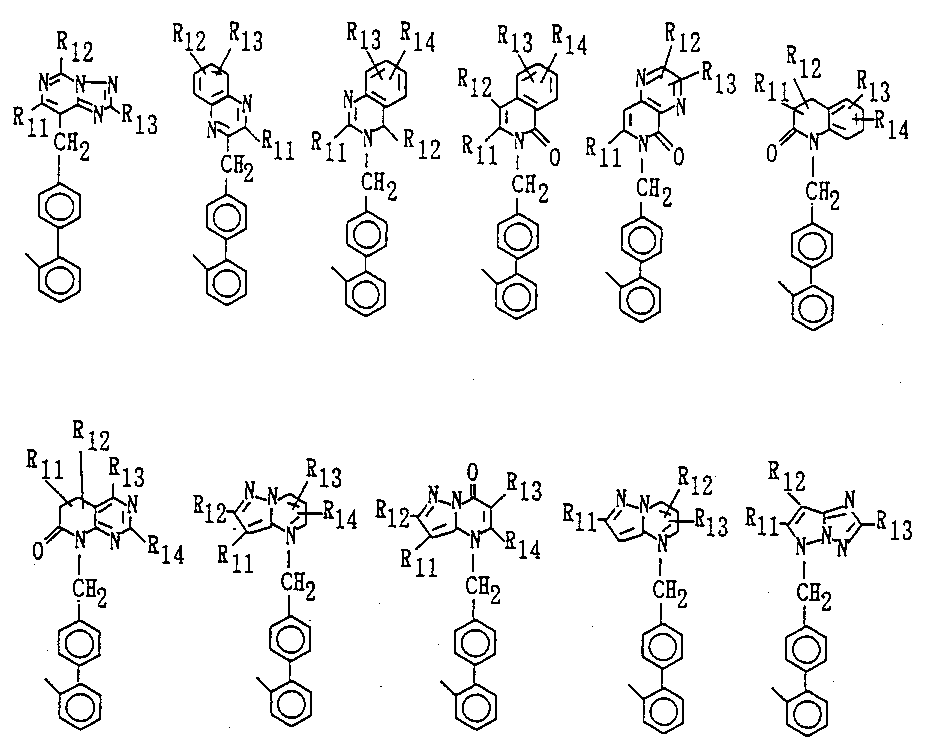 (where R₁₁, R₁₂, R₁₃ and R₁₄ are independently selected from the group consisting of hydrogen, a straight-chained or branched alkyl group having 1 - 4 carbon atoms, a hydroxyl group, a phenyl group, a halogen atom which is either one of F, Cl, Br and I, a trifluoroacetyl group, a pentafluoropropionyl group, a straight-chained or branched alkoxycarbonyl group having 1 - 4 carbon atoms, a phenyl-substituted straight-chained or branched alkyl group having 1 - 4 carbon atoms, and a hydroxyl-substituted straight-chained or branched alkyl group having 1 - 4 carbon atoms); and either one of the groups represented by the following formulae: 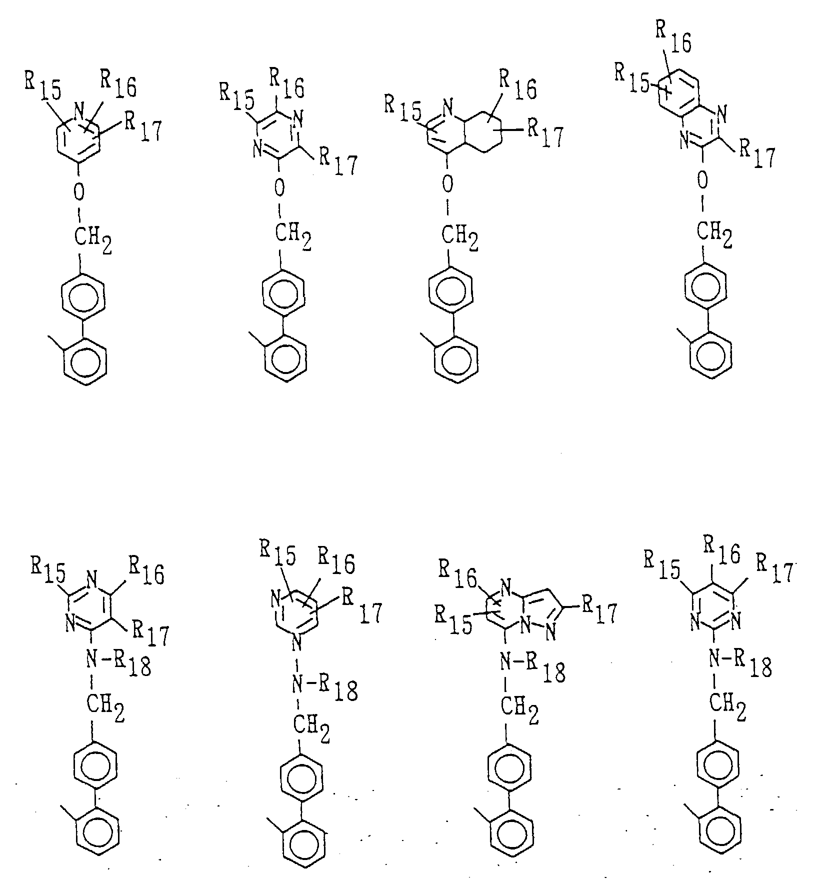 (where R₁₅, R₁₆, R₁₇ and R₁₈ are independently selected from the group consisting of hydrogen, a straight-chained or branched alkyl group having 1 - 4 carbon atoms, a hydroxyl group, a phenyl group, a halogen atom which is either one of F, Cl, Br and I, a trifluoroacetyl group, a pentafluoropropionyl group, a straight-chained or branched alkoxycarbonyl group having 1 - 4 carbon atoms, a phenyl-substituted straight-chained or branched alkyl group having 1 - 4 carbon atoms, and a hydroxyl-substituted straight-chained or branched alkyl group having 1 - 4 carbon atoms).
